(19) Europäisches Patentamt
European Patent Office
Office européen des brevets

(11) Veröffentlichungsnummer: **0 073 507**
**B1**

(12) # EUROPÄISCHE PATENTSCHRIFT

(45) Veröffentlichungstag der Patentschrift: **16.10.85**

(51) Int. Cl.⁴: **C 07 D 215/54**, A 61 K 31/64

(21) Anmeldenummer: **82107938.1**

(22) Anmeldetag: **28.08.82**

(54) **Sulfonylharnstoffe, Verfahren zu ihrer Herstellung, pharmazeutische Präparate auf Basis dieser Verbindungen und ihre Verwendung.**

(30) Priorität: **02.09.81 DE 3134780**

(43) Veröffentlichungstag der Anmeldung: **09.03.83 Patentblatt 83/10**

(45) Bekanntmachung des Hinweises auf die Patenterteilung: **16.10.85 Patentblatt 85/42**

(84) Benannte Vertragsstaaten: **AT BE CH DE FR GB IT LI LU NL SE**

(56) Entgegenhaltungen:
**AT - A - 357 560**
**DE - A - 2 114 629**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Hitzel, Volker, Dr., Kantstrasse 1b, D-6238 Hofheim am Taunus (DE)**
Erfinder: **Weyer, Rudi, Dr., Mozartstrasse 8, D-6233 Kelkheim (Taunus) (DE)**
Erfinder: **Geisen, Karl, Dr., Jahnstrasse 43, D-6000 Frankfurt am Main (DE)**
Erfinder: **Ritzel, Harald, Dr., An der Hayle 4, D-6500 Mainz (DE)**

**EP 0 073 507 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Sulfonylharnstoffe der Formel

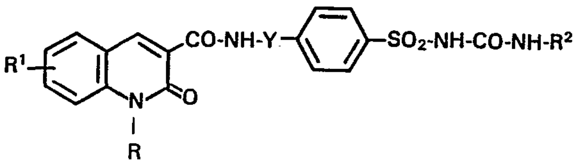

die als Substanz oder in Form ihrer physiologisch verträglichen Salze blutzuckersenkende Eigenschaften besitzen und sich durch eine starke Senkung des Blutzuckerspiegels auszeichnen und daher als Arzneimittel verwendet werden können.

In der Formel bedeuten:

Y Alkylen mit 2 - 3 C-Atomen,
R Alkyl mit 1 - 4 C-Atomen,
$R^1$ Wasserstoff, Chlor oder Methyl,
$R^2$ Alkyl mit 3 - 6 C-Atomen, Cycloalkyl, Alkylcycloalkyl, Cycloalkylalkyl, Cycloalkenyl, Alkylcycloalkenyl oder Cycloalkenylalkyl mit jeweils 5 - 8 C-Atomen.

In der Formel bedeutet

Y vorzugsweise $-CH_2-CH_2$, R vorzugsweise Methyl,
$R^1$ vorzugsweise Wasserstoff und $R^2$ vorzugsweise Cyclohexyl.

Die Erfindung betrifft ausserdem Verfahren zur Herstellung dieser Sulfonylharnstoffe, pharmazeutische Präparate, die diese enthalten oder aus ihnen bestehen sowie die Verwendung zur Behandlung des Diabetes.

Die Verfahren zur Herstellung der Sulfonylharnstoffe und ihrer Salze sind dadurch gekennzeichnet, dass man

a) mit der Gruppe

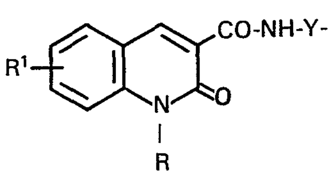

in 4-Stellung substituierte Benzolsulfonylcarbaminsäurederivate mit einem Amin $R^2$-$NH_2$ oder dessen Salzen umsetzt oder Sulfonamide der Formel

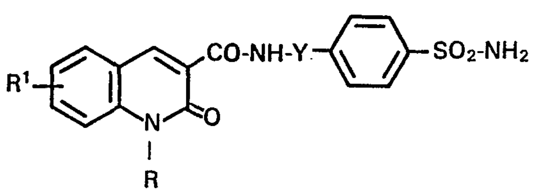

oder deren Salze mit einem $R^2$-substituierten Carbaminsäurederivat umsetzt,

b) mit der Gruppe

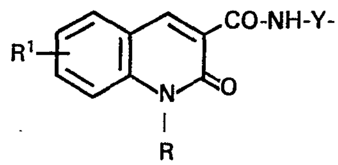

substituierte Benzolsulfonyl-isoharnstoffäther, -isothioharnstoffäther, -parabansäuren oder -halogenameisensäureamidine spaltet,

c) in

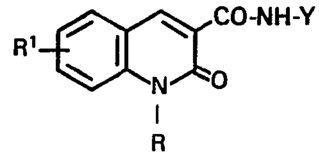

substituierten Benzolsulfonylthioharnstoffen das Schwefelatom durch Sauerstoff ersetzt,

d) entsprechende Benzolsulfinyl- oder -sulfenylharnstoffe oxydiert,

e) in Benzolsulfonylharnstoffe der Formel

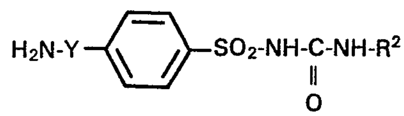

gegebenenfalls stufenweise den Rest

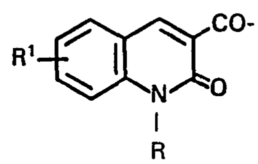

einführt,

f) entsprechend substituierte Benzolsulfonylhalogenide mit $R^2$-substituierten Harnstoffen oder deren Alkalisalzen umsetzt oder entsprechend substituierte Benzolsulfinsäure-halogenide oder, in Gegenwart von sauren Kondensationsmitteln, auch entsprechend substituierte Sulfinsäuren oder deren Alkalisalze, mit N-$R^2$-N'-hydroxyharnstoff umsetzt,

erhaltene Salze gegebenenfalls in die freien Verbindungen überführt oder die Reaktionsprodukte gegebenenfalls zur Salzbildung mit alkalischen Mitteln behandelt.

Die in Verfahren a) erwähnten Benzolsulfonylcarbaminsäurederivate sind z.B. Benzolsulfonylcarbaminsäureester, -thiolcarbaminsäureester, -harnstoffe, -semicarbazide oder -semicarbazone. Vorzugsweise wird die Umsetzung von Benzolsulfonylcarbaminsäureestern mit Aminen benutzt.

Die für die Umsetzung der in 4-Stellung substituierten Benzolsulfonamide verwendeten $R^2$-substituierten Carbaminsäurederivate sind z.B. die entsprechenden Isocyanate, Carbaminsäureester, Thiolcarbaminsäureester, Carbaminsäurehalogenide oder

Harnstoffe. Vorzugsweise verwendet man hiervon die Reaktion der Sulfonamide mit Isocyanaten bzw. Carbaminsäurechloriden.

Die erwähnten Benzolsulfonyl-carbaminsäureester bzw. -thiolcarbaminsäureester weisen in der Alkoholkomponente einen Alkylrest oder einen Arylrest oder auch einen heterocyclischen Rest auf. Da dieser Rest bei der Reaktion abgespalten wird, hat seine chemische Konstitution keinen Einfluss auf den Charakter des Endproduktes und kann deshalb in weiten Grenzen variiert werden. Das gleiche gilt für die N-$R^2$-substituierten Carbaminsäureester bzw. die entsprechenden Thiolcarbaminsäureester.

Die als Ausgangsstoffe des Verfahrens in Frage kommenden Benzolsulfonylharnstoffe sind an der der Sulfonylgruppe abgewandten Seite des Harnstoffmoleküls unsubstituiert oder ein- oder insbesondere zweifach substituiert. Da diese Substituenten bei der Reaktion mit Aminen abgespalten werden, kann ihr Charakter in weiten Grenzen variiert werden. Neben alkyl-, aryl-, acyl- oder heterocyclisch substituierten Benzolsulfonylharnstoffen eignen sich auch Benzolsulfonylcarbamoylimidazole und ähnliche Verbindungen oder Bisbenzolsulfonylharnstoffe, die an einem der Stickstoffatome noch einen weiteren Substituenten z.B. Methyl, tragen können. Man behandelt beispielsweise derartige Bis-(benzolsulfonyl)-harnstoffe oder auch N-Benzolsulfonyl-N'-acylharnstoffe mit $R^2$-substituierten Aminen und erhitzt die erhaltenen Salze auf erhöhte Temperaturen, insbesondere solche oberhalb 100°C.

Weiterhin eignen sich $R^2$-substituierte Harnstoffe oder solche $R^2$-substituierten Harnstoffe, die am freien Stickstoffatom noch ein- oder insbesondere zweifach substituiert sind als Ausgangsverbindungen zur Umsetzung mit

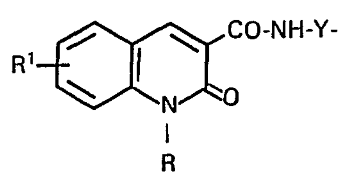

in 4-Stellung substituierten Benzolsulfonamiden. Als solche Ausgangsstoffe kommen beispielsweise in Frage N-Cyclohexyl-harnstoff, die entsprechenden N'-Acetyl, N'-Nitro, N'-Cyclohexyl, N',N'-Diphenyl- (wobei die beiden Phenylreste auch substituiert sowie direkt oder auch über ein Brückenglied wie $-CH_2-$, -NH-, -O- oder -S-miteinander verbunden sein können), N'-Methyl-N'-phenyl-, N,N-Dicyclohexylharnstoffe sowie Cyclohexyl-carbamoylimidazole, -pyrazole oder -triazole sowie solche der genannten Verbindungen, die anstelle des Cyclohexyls einen anderen im Bereich der Definition für $R^2$ liegenden Substituenten tragen.

Die Spaltung der als Ausgangsstoffe in Verfahren b) genannten Benzolsulfonylparabansäuren, -isoharnstoffäther, -isothioharnstoffäther oder -halogenameisensäureamidine erfolgt zweckmässig in Gegenwart von Basen. Isoharnstoffäther können auch in einem sauren Medium mit gutem Erfolg gespalten werden.

Der Ersatz des Schwefelatoms in der Thioharnstoffgruppierung von entsprechend substituierten Benzolsulfonylthioharnstoffen durch ein Sauerstoffatom erfolgt in bekannter Weise zum Beispiel mit Hilfe von Oxyden oder Salzen von Schwermetallen oder auch durch Anwendung von Oxydationsmitteln, wie Wasserstoffperoxid, Natriumperoxid, salpetriger Säure oder Permanganaten. Auch durch Behandlung mit Phosgen oder Phosphorpentachlorid werden Thioharnstoffe entschwefelt. Als Zwischenprodukte erhaltene Chlorameisensäureamidine bzw. Carbodiimide werden durch geeignete Massnahmen wie Verseifen oder Anlagerung von Wasser in die Benzolsulfonylharnstoffe überführt.

Die Oxidation von Benzolsulfinyl- bzw. Benzosulfenylharnstoffen erfolgt nach an sich bekannter Methode, vorzugsweise mit Oxydationsmitteln wie Permanganat oder Wasserstoffperoxid.

Die Acylierung der Sulfonylharnstoffe gemäss Verfahren e) wird mit reaktiven Derivaten der Säure

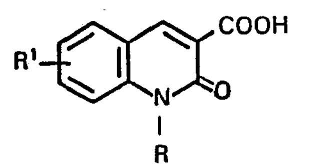

wie beispielsweise Halogeniden, gemischten Anhydriden oder Aktivestern durchgeführt.

Als Sulfonyl- bzw. Sulfinylhalogenide gemäss Verfahren f) eignen sich insbesondere die Chloride. Als saures Kondensationsmittel setzt man beispielsweise Thionylchlorid oder Polyphosphorsäure ein.

Die Herstellung der physiologisch verträglichen Salze erfolgt nach an sich bekannten Methoden. Zur Salzbildung sind insbesondere geeignet Alkali- und Erdalkalihydroxyde, -carbonate oder -bicarbonate sowie physiologisch verträgliche organische Basen.

Die Ausführungsformen des Verfahrens gemäss der Erfindung können im allgemeinen hinsichtlich der Reaktionsbedingungen weitgehend variiert und den jeweiligen Verhältnissen angepasst werden. Beispielsweise werden die Umsetzungen in Abwesenheit oder Anwesenheit von Lösungsmitteln, bei Raumtemperatur oder bei erhöhter Temperatur durchgeführt.

Je nach dem Charakter der Ausgangsstoffe kann das eine oder andere der beschriebenen Verfahren in einzelnen Fällen einen gewünschten individuellen Benzolsulfonylharnstoff nur in geringen Ausbeuten liefern oder zu dessen Synthese wenig geeignet sein. In solchen verhältnismässig selten auftretenden Fällen macht es dem Fachmann keine Schwierigkeiten, das gewünschte Produkt auf einem anderen der beschriebenen Verfahrenswege zu synthetisieren.

Die als Ausgangsstoffe dienenden 1-Substituierten-1,2-dihydro-2-oxo-chinolin-3-carbonsäuren sind literaturbekannt. Die entsprechenden 1,6-Disubstituierten-1,2-dihydro-2-oxo-chinolin-3-carbonsäuren können auf analoge Weise hergestellt werden.

Die erhaltenen Verbindungen werden vorzugsweise durch Umfällen und/oder Umkristallisieren gereinigt. Eine andere Methode zur Reinigung besteht

darin, dass man die Substanz aus einem kristallinen (Alkali)-Salz in einem geeigneten Lösungsmittel in Freiheit setzt.

Die erfindungsgemässen Verbindungen zeichnen sich durch wertvolle pharmakologische Eigenschaften, insbesondere blutzuckersenkende, aus. Sie eignen sich daher als Arzneimittel, insbesondere als Antidiabetika.

Die blutzuckersenkende Wirkung der erfindungsgemässen Benzolsulfonylharnstoffe wird beispielsweise dadurch festgestellt, dass man sie als freie Verbindungen oder in Form der Natriumsalze an normal ernährte Kaninchen verfüttert und den Blutzuckerwert nach der bekannten Methode von Hagedorn-Jensen oder mit einem Autoanalyzer über eine längere Zeitdauer ermittelt.

Die routinemässige Bestimmung der blutzuckersenkenden Wirkung erfolgt z.B. mit Dosierungen von z.B. 10 mg oder 2 mg oder 0,4 mg Wirksubstanz pro kg Versuchstier nach bekannten Methoden.

Die folgenden Verbindungen I bis IV wurden in Dosierungen von 2 mg/kg Kaninchen oral verabreicht und die Blutzuckerwerte wurden mit einem Autoanalyzer über eine längere Zeitdauer ermittelt. Die hierbei gemessene Blutzuckersenkung ist in der nachfolgenden Tabelle in % nach . . . Stunden angegeben.

I N-[4-⟨2-(1-Methyl-1,2-dihydro-2-oxo-chinolin-3-carboxamido)-äthyl⟩-benzolsulfonyl]-N'-cyclohexylharnstoff-Natriumsalz

II N-[4-⟨2-(1-Methyl-1,2-dihydro-2-oxo-6-chlor-chinolin-3-carboxamido)-äthyl⟩-benzolsulfonyl]-N'-butyl-harnstoff

III N-[4-⟨2-(1-Methyl-1,2-dihydro-2-oxo-chinolin-3-carboxamido)-äthyl⟩-benzolsulfonyl]-N'-cyclopentyl-harnstoff

IV N-[4-⟨2-(1-Methyl-1,2-dihydro-2-oxo-chinolin-3-carboxamido)-äthyl⟩-benzolsulfonyl]-N,-(3-methylcyclopentyl)-harnstoff

Tabelle

| Verbindung | Blutzuckersenkung am Kaninchen nach Verabreichung von 2 mg/kg p.o. in % nach | | | | |
|---|---|---|---|---|---|
| | 1 | 3 | 5 | 24 | 48 Stunden |
| I | 32 | 35 | 34 | 28 | 28 |
| II | 34 | 38 | 39 | 35 | 0 |
| III | 34 | 43 | 44 | 42 | 26 |
| IV | 26 | 31 | 34 | 37 | 5 |

Die erfindungsgemässen Benzolsulfonylharnstoffe zeichnen sich durch eine starke blutzuckersenkende Wirkung aus. Ausserdem sind die Verbindungen gut verträglich.

Die Eigenschaften der Verbindungen erlauben es, in der Therapie des Diabetes mellitus mit so geringen Dosen auszukommen, dass das Präparat nur die verminderte Ansprechbarkeit des Pankreas auf einen erhöhten Blutzuckerspiegel wieder normalisiert.

Die beschriebenen Sulfonylharnstoffe dienen vorzugsweise zur Herstellung von oral verabreichbaren Präparaten zur Behandlung des Diabetes mellitus. Sie werden als solche oder in Form ihrer Salze bzw. in Gegenwart von Stoffen, die zu einer Salzbildung führen, appliziert. Zur Salzbildung werden beispielsweise alkalische Mittel wie Alkali- oder Erdalkalihydroxyde, -carbonate oder -bicarbonate herangezogen. Die Präparate können neben dem Sulfonylharnstoff bzw. dessen Salz auch noch andere Wirkstoffe enthalten. Als medizinische Präparate kommen vorzugsweise Tabletten in Betracht, die neben den Sulfonylharnstoffen oder deren Salzen die üblichen Träger- und Hilfsstoffe wie Talkum, Stärke, Milchzucker oder Magnesiumstearat enthalten. Dabei kann es zweckmässig sein, den oder die Wirkstoffe in gemahlener oder fein gefällter Form oder als Gemisch dieser Formen einzusetzen. Ein Präparat, das die erfindungsgemässen Benzolsulfonylharnstoffe als Wirkstoff enthält, z.B. eine Tablette oder ein Pulver mit oder ohne Zusätze, ist zweckmässig in eine geeignet dosierte Form gebracht. Als Dosis ist dabei eine solche zu wählen, die der Wirksamkeit des verwendeten Benzolsulfonylharnstoffs und dem gewünschten Effekt angepasst ist. Zweckmässig beträgt die Dosierung je Einheit etwa 0,5 bis 50 mg, vorzugsweise 1 bis 20 mg, jedoch können auch darüber oder darunter liegende Dosierungseinheiten verwendet werden, die gegebenenfalls vor Applikation zu teilen bzw. zu vervielfachen sind.

Die nachfolgenden Beispiele zeigen einige der zahlreichen Verfahrensvarianten, die zur Synthese der erfindungsgemässen Sulfonylharnstoffe geeignet sind. Sie sollen jedoch nicht eine Einschränkung des Erfindungsgegenstandes darstellen.

*Beispiel 1*

*N-[4-⟨2-(1-Methyl-1,2-dihydro-2-oxo-chinolin-3-carboxamido)-äthyl⟩-benzolsulfonyl]-N'-cyclohexylharnstoff-Kaliumsalz*

38,54 g 4-[2-(1-Methyl-1,2-dihydro-2-oxo-chinolin-3-carboxamido)-äthyl]-benzolsulfonamid [Schmp. 235 - 237°C, hergestellt durch Reaktion von 1-Methyl-1,2-dihydro-2-oxo-chinolin-carbonsäure mit Chlorameisensäuremethylester und Triäthylamin und anschliessende Umsetzung mit 4-(2-Aminoäthyl)-benzolsulfonamid] werden in 2 l Butanon-2 suspendiert. Nach Zugabe von 27,6 g gemahlener, wasserfreier Pottasche und 14,0 ml Cyclohexylisocyanat wird 5 Stunden unter Rückfluss gerührt. Nach dem Erkalten saugt man die Kaliumsalze ab und trocknet sie. Anschliessend wird in 50 ml Wasser suspendiert und die Mischung unter Rühren mit 2N Salzsäure auf pH 7,5 gebracht. Man saugt erneut ab und kocht mit 500 ml Äthanol aus. Das so erhaltene N-[4-⟨2-(1-Methyl-1,2-dihydro-2-oxo-chinolin-3-carboxamido)-äthyl⟩-benzolsulfonyl]-N'-cyclohexylharnstoff-Kaliumsalz schmilzt nach dem Trocknen bei 281 - 283°C.

In analoger Weise erhält man das

N-[4-⟨2-(1-Methyl-1,2-dihydro-2-oxo-chinolin-3-carboxamido)-äthyl⟩-benzolsulfonyl]-N'-$\Delta^3$-cyclohexenyl-harnstoff-Kaliumsalz von Schmp. 259 bis 261°C (aus Äthanol-Dimethylformamid)

N-[4-⟨2-(1-Methyl-1,2-dihydro-2-oxo-chinolin-3-

-carboxamido)-äthyl>-benzolsulfonyl]-N'-cyclooctyl-harnstoff-Kaliumsalz vom Schmp. 262 - 264°C (aus Äthanol)

*Beispiel 2*

*N-[4-<2-(1-Methyl-1,2-dihydro-2-oxo-chinolin-3-carboxamido)-äthyl>-benzolsulfonyl]-N'-cyclohexylharnstoff*

8,7 g 4-[2-(1-Methyl-1,2-dihydro-2-oxo-chinolin-3-carboxamido)-äthyl]-benzolsulfonamid (hergestellt wie in Beispiel 1 angegeben) werden in 100 ml Aceton und 11,25 ml 2N Natronlauge suspendiert. Nach dem Abkühlen auf 0 - 5°C tropft man eine Lösung von 3,1 g Cyclohexylisocyanat in 10 ml Aceton zu. Man rührt 1 Stunde bei 0 - 5°C und 4 Stunden bei Raumtemperatur nach und bringt den Niederschlag durch Verdünnen mit Wasser in Lösung. Nach dem Filtrieren wird mit 2N Salzsäure angesäuert, der Niederschlag abgesaugt und aus Äthanol/Dimethylformamid umkristallisiert. Der Schmelzpunkt des N-[4-<2-(1-Methyl-1,2-dihydro-2-oxo-chinolin-3-carboxamido)-äthyl>-benzolsulfonyl]-N'-cyclohexylharnstoffs liegt bei 213 - 215°C.

*Beispiel 3*

*N-[4-<2-(1-Methyl-1,2-dihydro-2-oxo-chinolin-3-carboxamido)-äthyl>-benzolsulfonyl]-N'-cyclohexylharnstoff-Natriumsalz*

4,0 g des nach Beispiel 2 erhaltenen N-[4-<2-(1-Methyl-1,2-dihydro-2-oxo-chinolin-3-carboxamido)-äthyl>-benzolsulfonyl]-N'-cyclohexylharnstoffs werden in eine Mischung von 100 ml Methanol und 3,91 ml 2N Natronlauge portionsweise eingetragen. Nach kurzer Nachrührzeit fällt das kristalline Natriumsalz aus. Man saugt ab, behandelt mit heissem Äthanol und trocknet. Der Schmelzpunkt liegt bei 291 - 293°C.

*Beispiel 4*

*N-[4-<2-(1-Methyl-1,2-dihydro-2-oxo-chinolin-3-carboxamido)-äthyl>-benzolsulfonyl]-N'-(4-methylcyclohexyl)-harnstoff*

2,9 g 4-[2-(1-Methyl-1,2-dihydro-2-oxo-chinolin-3-carboxamido)-äthyl]-benzolsulfonamid (hergestellt wie in Beispiel 1 angegeben) werden in 150 ml Butanon-2 zusammen mit 2,07 g gemahlener, wasserfreier Pottasche suspendiert. Nach Zusatz von 1,25 g 4-Methylcyclohexylisocyanat wird 4 Stunden unter Rückfluss gerührt. Die erkaltete Mischung wird abgesaugt, der Niederschlag in 150 ml Wasser suspendiert und mit verdünnter Salzsäure angesäuert. Der ausgefällte N-[4-<2-(1-Methyl-1,2-dihydro-2-oxo-chinolin-3-carboxamido)-äthyl>-benzolsulfonyl]-N'-(4-methylcyclohexyl)-harnstoff wird abgesaugt, mit Äthanol ausgekocht und getrocknet. Er schmilzt bei 221 - 223°C.

In analoger Weise erhält man den

N-[4-<2-(1-Methyl-1,2-dihydro-2-oxo-chinolin-3-carboxamido)-äthyl>-benzolsulfonyl]-N'-cyclopentyl-harnstoff vom Schmp. 197 - 199°C (aus Äthanol-Dimethylformamid)

N-[4-<2-(1-Methyl-1,2-dihydro-2-oxo-chinolin-3-carboxamido)-äthyl>-benzolsulfonyl]-N'-cyclopentylmethyl-harnstoff vom Schmp. 218 - 220°C (aus Äthanol-Dimethylformamid)

N-[4-<2-(1-Methyl-1,2-dihydro-2-oxo-chinolin-3-carboxamido)-äthyl>-benzolsulfonyl]-N'-cycloheptyl-harnstoff vom Schmp. 209 - 211°C (aus Äthanol-Dimethylformamid)

N-[4-<2-(1-Methyl-1,2-dihydro-2-oxo-chinolin-3-carboxamido)-äthyl>-benzolsulfonyl]-N'-(3-methylcyclopentyl)-harnstoff vom Schmp. 204 - 206°C (aus Äthanol-Dimethylformamid)

N-[4-<2-(1-Methyl-1,2-dihydro-2-oxo-chinolin-3-carboxamido)-äthyl>-benzolsulfonyl]-N'-hexyl-harnstoff vom Schmp. 202 - 204°C (aus Äthanol-Dimethylformamid)

N-[4-<2-(1-Methyl-1,2-dihydro-2-oxo-chinolin-3-carboxamido)-äthyl>-benzolsulfonyl]-N'-propyl-harnstoff vom Schmp. 203 - 205°C (aus Äthanol-Dimethylformamid)

In analoger Weise erhält man aus 4-[2-(1-Äthyl-1,2-dihydro-2-oxo-chinolin-3-carboxamido)-äthyl]-benzolsulfonamid (Schmp. 199 - 200°C, hergestellt analog Beispiel 1) den N-[4-<2-(1-Äthyl-1,2-dihydro-2-oxo-chinolin-3-carboxamido)-äthyl>-benzolsulfonyl]-N'-cyclohexyl-harnstoff vom Schmp. 157 - 158°C (aus Äthanol-Dimethylformamid)

In analoger Weise erhält man aus 4-[2-(1-Butyl-1,2-dihydro-2-oxo-chinolin-3-carboxamido)-äthyl]-benzolsulfonamid (Schmp. 290 - 294°C, hergestellt analog Beispiel 1) den N-[4-<2-(1-Butyl-1,2-dihydro-2-oxo-chinolin-3-carboxamido)-äthyl>-benzolsulfonyl]-N'-cyclohexyl-harnstoff vom Schmp. 179 - 181°C (aus Äthanol)

*Beispiel 5*

*N-[4-<2-(1-Methyl-1,2-dihydro-2-oxo-chinolin-3-carboxamido)-äthyl>-benzolsulfonyl]-N'-cyclohexyl-harnstoff*

2,5 g 4-[2-(1-Methyl-1,2-dihydro-2-oxo-6-chlor-chinolin-3-carboxamido)-äthyl]-benzolsulfonamid [Schmp. 237 - 239°C, hergestellt in analoger Weise wie in Beispiel 1 angegeben aus 1-Methyl-1,2-dihydro-2-oxo-6-chlor-chinolin-3-carbonsäure und 4-(2-Aminoäthyl)-benzolsulfonamid] werden in 150 ml Butanon-2 suspendiert.

Nach Zusatz von 1,66 g wasserfreier, gemahlener Pottasche und 0,82 g Cyclohexylisocyanat wird 4 Stunden unter Rückfluss gerührt. Nach dem Abkühlen wird abfiltriert, der Rückstand in Wasser suspendiert und die Mischung mit verdünnter Salzsäure angesäuert. Der so ausgefällte N-[4-<2-(1-Methyl-1,2-dihydro-2-oxo-6-chlor-chinolin-3-carboxamido)-äthyl>-benzolsulfonyl]-N'-cyclohexyl-harnstoff wird aus Äthanol-Dimethylformamid umkristallisiert und schmilzt bei 209 - 210°C.

In analoger Weise erhält man den N-[4-<2-(1-Methyl-1,2-dihydro-2-oxo-6-chlor-chinolin-3-carboxamido)-äthyl>-benzolsulfonyl]-N'-butyl-harnstoff vom Schmp. 157 - 158°C (aus Methanol-Dimethylformamid)

*Beispiel 6*

*N-[4-⟨2-(1,6-Dimethyl-1,2-dihydro-2-oxo-chinolin-3-carboxamido)-äthyl⟩-benzolsulfonyl]-N'-cyclohexyl-harnstoff*

3,0 g 4-[2-(1,6-Dimethyl-1,2-dihydro-2-oxo-chinolin-3-carboxamido)-äthyl]-benzolsulfonamid [Schmp. 234 - 236°C, hergestellt in analoger Weise wie in Beispiel 1 angegeben aus 1,6-Dimethyl-1,2-dihydro-2-oxo-chinolin-3-carbonsäure und 4-(2-Aminoäthyl)-benzolsulfonamid] werden zusammen mit 2,07 g wasserfreier, gemahlener Pottasche in 150 ml Butanon-2 und 1,03 g Cyclohexylisocyanat 4 Stunden unter Rückfluss gerührt. Nach dem Abkühlen filtriert man ab, löst die Salze in Wasser und säuert mit 2N Salzsäure an. Der ausgefällte N-[4-⟨2-(1,6-Dimethyl-1,2-dihydro-2-oxo-chinolin-3-carboxamido)-äthyl⟩-benzolsulfonyl]-N'-cyclohexyl-harnstoff wird aus Nitromethan umkristallisiert und schmilzt bei 226 - 228°C.

In analoger Weise erhält man den

N-[4-⟨2-(1,6-Dimethyl-1,2-dihydro-2-oxo-chinolin-3-carboxamido)-äthyl⟩-benzolsulfonyl]-N'-butyl-harnstoff vom Schmp. 194 - 196°C (aus Nitromethan)

N-[4-⟨2-(1,6-Dimethyl-1,2-dihydro-2-oxo-chinolin-3-carboxamido)-äthyl⟩-benzolsulfonyl]-N'-(4-methylcyclohexyl)-harnstoff vom Schmp. 203 - 205°C (aus Nitromethan)

*Beispiel 7*

*N-[4-⟨2-(1-Methyl-1,2-dihydro-2-oxo-chinolin-3-carboxamido)-äthyl⟩-benzolsulfonyl]-N'-cyclohexyl-harnstoff*

0,8 g N-[4-⟨2-(1-Methyl-1,2-dihydro-2-oxo-chinolin-3-carboxamido)-äthyl⟩-benzolsulfonyl]-N'-cyclohexyl-thioharnstoff (Schmp. 208 - 210°C, hergestellt analog Beispiel 4 unter Verwendung von Cyclohexylsenföl) werden in 100 ml Wasser und 100 ml Methanol mit 0,32 g gelbem Quecksilberoxid 5 Stunden bei 60°C gerührt. Nach dem Filtrieren engt man das Filtrat ein und kristallisiert den Rückstand aus Äthanol-Dimethylformamid um. Der Schmelzpunkt des N-[4-⟨2-(1-Methyl-1,2-dihydro-2-oxo-chinolin-3-carboxamido)-äthyl⟩-benzolsulfonyl]-N'-cyclohexylharnstoffs liegt bei 213 - 215°C.

*Beispiel 8*

*N-[4-⟨2-(1-Methyl-1,2-dihydro-2-oxo-chinolin-3-carboxamido)-äthyl⟩-benzolsulfonyl]-N'-cyclohexyl-harnstoff*

0,4 g N-[4-⟨2-(1-Methyl-1,2-dihydro-2-oxo-chinolin-3-carboxamido)-äthyl⟩-benzolsulfonyl]-N'-cyclohexyl-thioharnstoff (Schmp. 208 - 210°C, hergestellt analog Beispiel 4 unter Verwendung von Cyclohexylsenföl) und 0,16 g gelbes Quecksilberoxid werden in 30 ml Methanol 5 Stunden bei 50 - 55°C gerührt. Nach dem Filtrieren dampft man das Filtrat ein und erhält den N-[4-⟨2-(1-Methyl-1,2-dihydro-2-oxo-chinolin-3-carboxamido)-äthyl⟩-benzolsulfonyl]-N'-cyclohexyl-isoharnstoffmethyläther als Rückstand.

Der Isoharnstoffmethyläther wird mit 5 ml conc. Salzsäure und 10 ml Dioxan einige Minuten auf dem Dampfbad erwärmt. Durch Verdünnen mit Eiswasser wird der N-[4-⟨2-(1-Methyl-1,2-dihydro-2-oxo-chinolin-3-carboxamido)-äthyl⟩-benzolsulfonyl]-N'-cyclohexyl-harnstoff ausgefällt. Er wird umkristallisiert aus Äthanol-Dimethylformamid und schmilzt bei 213 - 215°C.

Die in Beispiel 5 verwendete 1-Methyl-1,2-dihydro-2-oxo-6-chlor-chinolin-3-carbonsäure (Schmp. 258 - 260°C) wird erhalten durch Methylierung von 1,2-Dihydro-2-oxo-6-chlor-chinolin-3-carbonsäure (Schmp. > 310°C) mit Dimethylsulfat. Letztere ist aus 5-Chlor-2-nitro-benzalmalonsäure durch reduktive Cyclisierung mit $FeSO_4$ in ammoniakalischer Lösung zugänglich.

Die in Beispiel 6 verwendete 1,6-Dimethyl-1,2-dihydro-2-oxo-chinolin-3-carbonsäu-re (Schmp. 238 - 240°C) wird hergestellt aus 1,2-Dihydro-2-oxo-6-methyl-chinolin-3-carbonsäure (Schmp. 320°C) und Dimethylsulfat. Die 1,2-Dihydro-2-oxo-6-methyl-chinolin-3-carbonsäure erhält man aus 5-Methyl-2-nitro-benzalmalonsäure (Schmp. 180 - 182°C) und $FeSO_4$ in ammoniakalischer Lösung.

*Beispiel 9*

*N-[4-⟨2-(1-Methyl-1,2-dihydro-2-oxo-chinolin-3-carboxamido)-äthyl⟩-benzolsulfonyl]-N'-cyclohexyl-harnstoff*

2,0 g 4-[2-(1-Methyl-1,2-dihydro-2-oxo-chinolin-3-carboxamido)-propyl]-benzolsulfonamid [vom Schmp. 199 - 200°C, erhalten durch Umsetzung von 1-Methyl-1,2-dihydro-2-oxo-chinolin-3-carbonsäure und 4-(2-Amino-propyl)-benzolsulfonamid analog Beispiel 1] werden mit 1,38 g Kaliumcarbonat (gemahlen) in 100 ml Butanon-(2) während 4 Stunden mit 0,68 g Cyclohexylisocyanat unter Rückfluss gerührt. Nach dem Abkühlen saugt man die ausgefallenen Salze ab, löst diese in 100 ml $H_2O$ und säuert mit 2N Salzsäure an. Der zuerst etwas ölig anfallende N-[4-⟨2-(1-Methyl-1,2-dihydro-2-oxo-chinolin-3-carboxamido)-äthyl⟩-benzolsulfonyl]-N'-cyclohexyl-harnstoff wird aus Nitromethan und 2mal aus Äthanol umkristallisiert und schmilzt bei 183 bis 185°C.

In analoger Weise erhält man den N-[4-⟨2-(1-Methyl-1,2-dihydro-2-oxo-chinolin-3-carboxamido)-propyl⟩-benzolsulfonyl]-N'-(4-methyl-cyclohexyl)-harnstoff vom Schmp. 138 - 140°C (aus Äthanol)

N-[4-⟨2-(1-Methyl-1,2-dihydro-2-oxo-chinolin-3-carboxamido)-propyl⟩-benzolsulfonyl]-N'-butyl-harnstoff vom Schmp. 154 - 155°C (aus Nitromethan).

**Patentansprüche für die Vertragsstaaten:**
BE, CH, DE, FR, GB, IT, LI, LU, NL, SE

1. Sulfonylharnstoffe der Formel

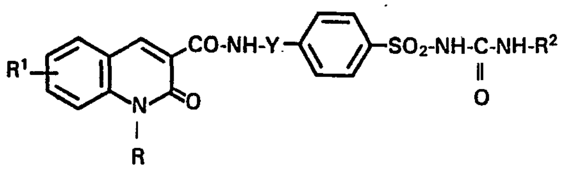

in welcher bedeuten:

Y Alkylen mit 2 - 3 C-Atomen

R Alkyl mit 1 - 4 C-Atomen

$R^1$ Wasserstoff, Chlor oder Methyl

$R^2$ Alkyl mit 3 - 6 C-Atomen, Cycloalkyl, Alkylcycloalkyl, Cycloalkylalkyl, Cycloalkenyl, Alkylcycloalkenyl oder Cycloalkenylalkyl mit jeweils 5 - 8 C-Atomen

und deren physiologisch verträglichen Salze.

2. Verfahren zur Herstellung von Sulfonylharnstoffen gemäss Anspruch 1, dadurch gekennzeichnet, dass man

a) mit der Gruppe

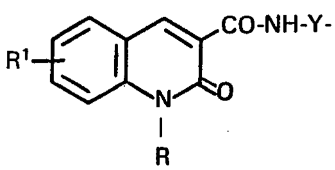

in 4-Stellung substituierte Benzolsulfonylcarbaminsäurederivate mit einem Amin $R^2$-$NH_2$ oder dessen Salzen umsetzt

oder Sulfonamide der Formel

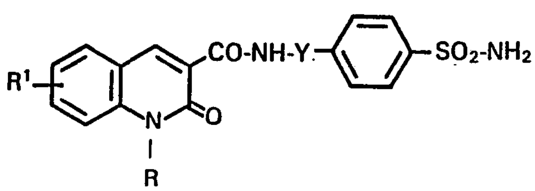

oder deren Salze mit einem $R^2$-substituierten Carbaminsäurederivat umsetzt,

b) mit der Gruppe

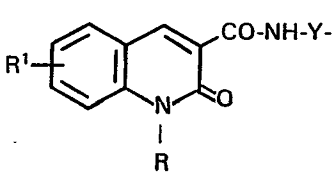

substituierte Benzolsulfonyl-isoharnstoffäther, -isothioharnstoffäther, -parabansäuren oder -halogenameisensäureamidine spaltet,

c) in

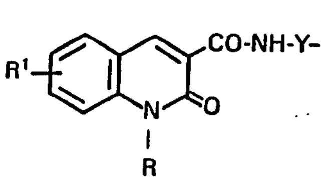

substituierten Benzolsulfonylthioharnstoffen das Schwefelatom durch Sauerstoff ersetzt,

d) entsprechende Benzolsulfinyl- oder -sulfenylharnstoffe oxydiert,

e) in Benzolsulfonylharnstoffe der Formel

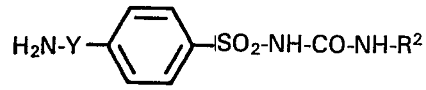

gegebenenfalls stufenweise den Rest

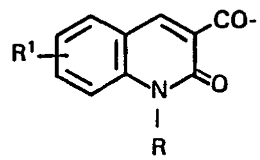

einführt,

f) entsprechend substituierte Benzolsulfonylhalogenide mit $R^2$-substituierten Harnstoffen oder deren Alkalisalzen umsetzt oder entsprechend substituierte Benzolsulfinsäure-halogenide oder, in Gegenwart von sauren Kondensationsmitteln, auch entsprechend substituierte Sulfinsäuren oder deren Alkalisalze, mit N-$R^2$-N'-hydroxyharnstoff umsetzt,

erhaltene Salze gegebenenfalls in die freien Verbindungen überführt oder die Reaktionsprodukte gegebenenfalls zur Salzbildung mit alkalischen Mitteln behandelt.

3. Arzneimittel auf Basis eines Sulfonylharnstoffs gemäss Anspruch 1 oder eines seiner Salze.

4. Sulfonylharnstoff gemäss Anspruch 1 oder eines seiner Salze zur Bekämpfung von Diabetes.

5. Verfahren zur Herstellung eines Arzneimittels gemäss Anspruch 3, dadurch gekennzeichnet, dass man einen Sulfonylharnstoff der im Anspruch 1 angegebenen Formel oder eines seiner Salze in eine geeignete Applikationsform bringt.

**Patentansprüche für den Vertragsstaat:** AT

1. Verfahren zur Herstellung eines Sulfonylharnstoffes der Formel

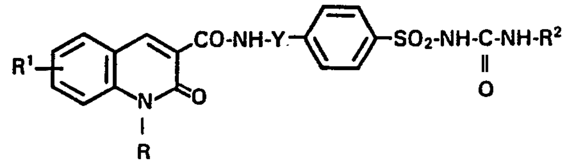

in welcher bedeuten:

Y Alkylen mit 2 - 3 C-Atomen

R Alkyl mit 1 - 4 C-Atomen

$R^1$ Wasserstoff, Chlor, Methyl

$R^2$ Alkyl mit 3 - 6 C-Atomen, Cycloalkyl, Alkylcycloalkyl, Cycloalkylalkyl, Cycloalkenyl, Alkylcycloalkenyl oder Cycloalkenylalkyl mit jeweils 5 - 8 C-Atomen

und seiner physiologisch verträglichen Salze, dadurch gekennzeichnet, dass man

a) mit der Gruppe

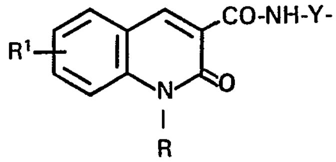

in 4-Stellung substituierte Benzolsulfonylcarbaminsäurederivate mit einem Amin $R^2$-$NH_2$ oder dessen Salzen umsetzt oder Sulfonamide der Formel

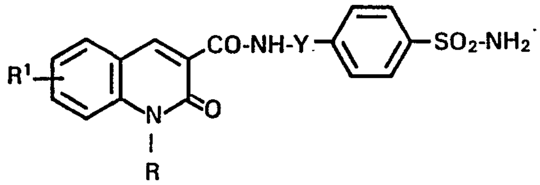

oder deren Salze mit einem $R^2$-substituierten Carbaminsäurederivat umsetzt,

b) mit der Gruppe

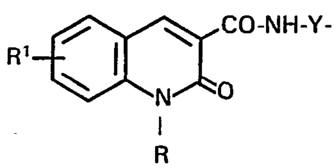

substituierte Benzolsulfonyl-isoharnstoffäther, -isothioharnstoffäther, -parabansäuren oder -halogenameisensäureamidine spaltet,

c) in

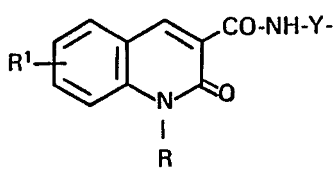

substituierten Benzolsulfonylthioharnstoffen das Schwefelatom durch Sauerstoff ersetzt,

d) entsprechende Benzolsulfinyl- oder -sulfenylharnstoffe oxydiert,

e) in Benzolsulfonylharnstoffe der Formel

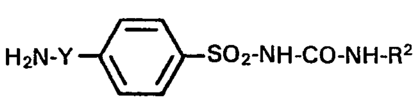

gegebenenfalls stufenweise den Rest

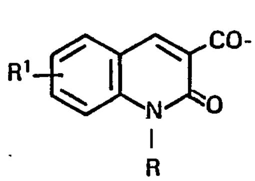

einführt,

f) entsprechend substituierte Benzolsulfonylhalogenide mit $R^2$-substituierten Harnstoffen oder deren Alkalisalzen umsetzt oder entsprechend substituierte Benzolsulfinsäure-halogenide oder, in Gegenwart von sauren Kondensationsmitteln, auch entsprechend substituierte Sulfinsäuren oder deren Alkalisalze, mit N-$R^2$-N'-hydroxyharnstoff umsetzt,

erhaltene Salze gegebenenfalls in die freien Verbindungen überführt oder die Reaktionsprodukte gegebenenfalls zur Salzbildung mit alkalischen Mitteln behandelt.

2. Verfahren zur Herstellung eines Arzneimittels, dadurch gekennzeichnet, dass man einen Sulfonylharnstoff der im Anspruch 1 angegebenen Formel oder eines seiner Salze gegebenenfalls unter Zusatz von üblichen Träger- und Hilfsstoffen in eine geeignete Applikationsform bringt.

**Claims for the Contracting states:**
BE, CH, DE, FR, GB, IT, LI, LU, NL, SE

1. A sulfonylurea of the formula

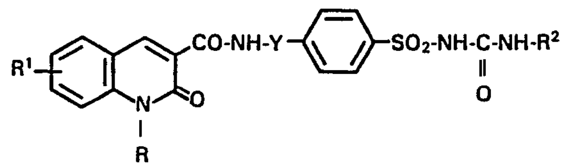

in which Y denotes alkylene having 2-3 C-atoms, R denotes alkyl having 1-4 C-atoms, $R^1$ denotes hydrogen, chlorine or methyl and $R^2$ denotes alkyl having 3-6 C-atoms, cycloalkyl, alkylcycloalkyl, cycloalkylalkyl, cycloalkenyl, alkylcycloalkenyl or cycloalkenylalkyl, each having 5-8 C-atoms, and its physiologically tolerated salts.

2. Processes for the preparation of sulfonylureas as claimed in claim 1 which comprise

a) reacting benzenesulfonylcarbamic acid derivatives substituted in the 4-position with the group

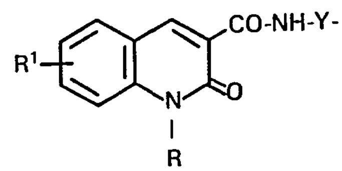

with an amine $R^2$-$NH_2$ or its salts or reacting sulfonamides of the formula

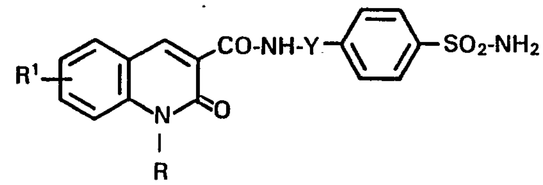

or their salts with a carbamic acid derivative substituted by $R^2$,

b) cleaving benzenesulfonyl-isourea ethers, -isothiourea ethers, -parabanic acids or -halogenoformamidines substituted with the group

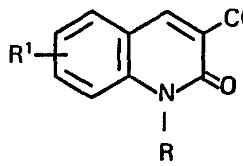

c) replacing the sulfur atom by oxygen in benzenesulfonylthioureas substituted by

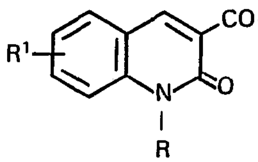

d) oxidizing correspondingly substituted benzenesulfinyl- or -sulfenyl-ureas

e) introducing the radical

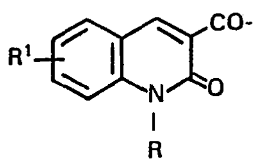

into benzenesulfonylureas of the formula

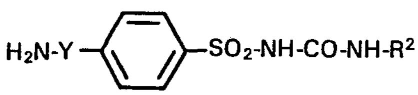

optionally in steps,

f) reacting correspondingly substituted benzenesulfonyl halides with $R^2$-substituted ureas or their alkali metal salts or reacting correspondingly substituted benzenesulfinyl halides or, in the presence of acid condensing agents, correspondingly substituted sulfinic acids or their alkali metal salts, with N-$R^2$-N'-hydroxyurea,

optionally converting the salts obtained into the free compounds or optionally treating the reaction products with alkaline agents to form salts.

3. A medicament based on a sulfonylurea as claimed in claim 1 or on one of its salts.

4. A sulfonylurea as claimed in claim 1 or one of its salts for controlling diabetes.

5. A process for the preparation of a medicament as claimed in claim 3 which comprises converting a sulfonylurea of the formula indicated in claim 1 or one of its salts into a suitable form for administration.

**Claims for the Contracting state:** AT

1. Processes for the preparation of a sulfonylurea of the formula

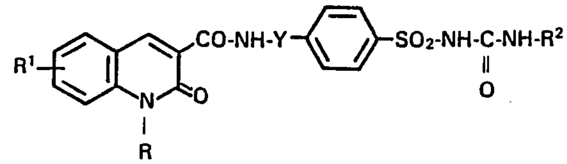

in which Y denotes alkylene having 2-3 C-atoms, R denotes alkyl having 1-4 C-atoms, $R^1$ denotes hydrogen, chlorine or methyl and $R^2$ denotes alkyl having 3-6 C-atoms, cycloalkyl, alkylcycloalkyl, cycloalkylalkyl, cycloalkenyl, alkylcycloalkenyl or cycloalkenylalkyl, each having 5-8 C-atoms, and of its physiologically tolerated salts which comprise

a) reacting benzenesulfonylcarbamic acid derivatives substituted in the 4-position with the group

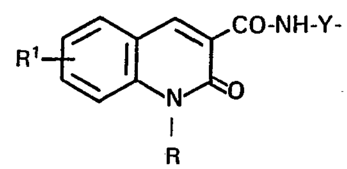

with an amine $R^2$-$NH_2$ or its salts or reacting sulfonamides of the formula

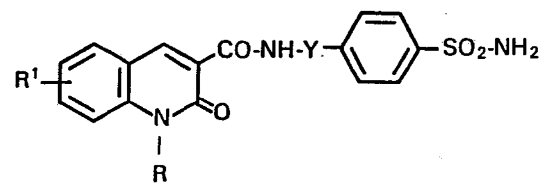

or their salts with a carbamic acid derivative substituted by $r^2$,

b) cleaving benzenesulfonyl-isourea ethers, -isothiourea ethers, -parabanic acids or -halogenoformamidines substituted with the group

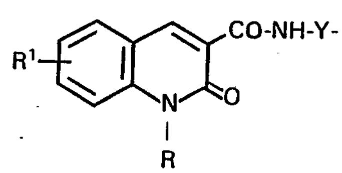

c) replacing the sulfur atom by oxygen in benzenesulfonylthioureas substituted by

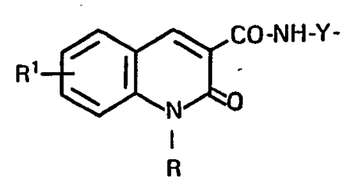

d) oxidizing correspondingly substituted benzenesulfinyl- or -sulfenyl-ureas

e) introducing the radical

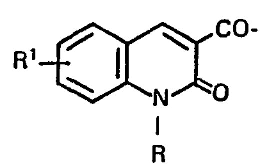

into benzenesulfonylureas of the formula

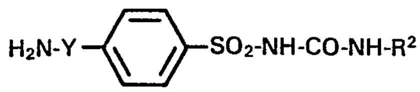

optionally in steps,

f) reacting correspondingly substituted benzenesulfonyl halides with $R^2$-substituted ureas or their alkali metal salts or reacting correspondingly substituted benzenesulfinyl halides or, in the presence of acid condensing agents, correspondingly substituted sulfinic acids or their alkali metal salts, with N-$R^2$-N'-hydroxyurea, optionally converting the salts obtained into the free compounds or optionally treating the reaction products with alkaline agents to form salts.

2. A process for the preparation of a medicament which comprises converting a sulfonylurea of the formula indicated in claim 1 or one of its salts, optionally by the addition of customary carriers or auxiliaries, into a suitable form for administration.

**Revendications pour les Etats contractants:**
BE, CH, DE, FR, GB, IT, LI, LU, NL, SE

1. Sulfonylurées de formule

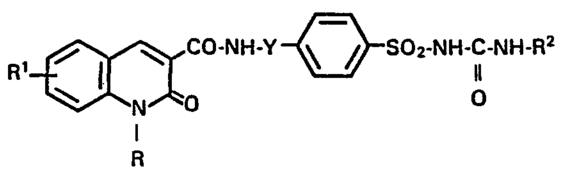

dans laquelle
Y représente un groupe alkylène ayant de 2 à 3 atomes de carbone
R représente un groupe alkyle ayant de 1 à 4 atomes de carbone,
$R^1$ représente l'hydrogène, le chlore, un groupe méthyle,
$R^2$ représente un groupe alkyle ayant de 3 à 6 atomes de carbone, cycloalkyle, alkylcycloalkyle, cycloalkylalkyle, cycloalcényle, alkylcycloalcényle ou cycloalcénylalkyle, ayant chacun de 5 à 8 atomes de carbone,
et leurs sels physiologiquement acceptables.

2. Procédé pour la préparation de sulfonylurées selon la revendication 1, caractérisé en ce que

a) on fait réagir des dérivés de l'acide benzènesulfonylcarbamique substitué en position 4 avec le groupe

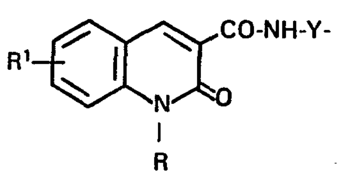

avec une amine $R^2$-$NH_2$ ou ses sels,

ou on fait réagir des sulfonamides de formule

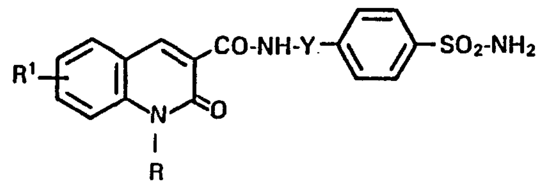

ou leurs sels avec un dérivé de l'acide carbamique $R^2$-substitué,

b) on scinde des éthers de benzènesulfonyl-isourées, des éthers de benzènesulfonyl-isothiourées, des acides benzènesulfonyl-parabaniques ou des amidines d'acides benzènesulfonyl-halogénoformiques, tous substitués avec le groupe

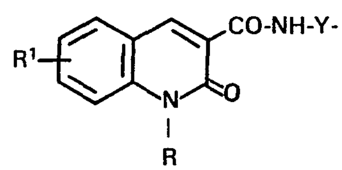

c) dans des benzènesulfonylthiourées substituées avec le groupe

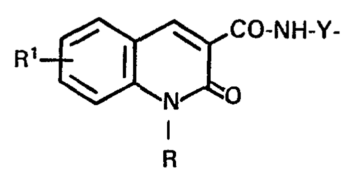

on remplace l'atome de soufre par de l'oxygène,

d) on oxyde des benzènesulfinyl- ou benzène sulfényl-urées correspondantes;

e) dans des benzènesulfonylurées de formule

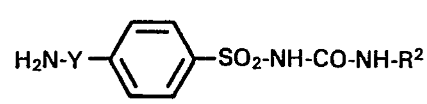

on introduit éventuellement par étapes le radical

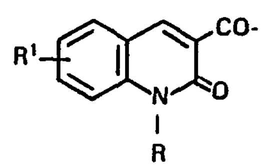

f) on fait réagir des halogénures de benzènesulfonyle substitués correspondants avec des urées $R^2$-substituées ou avec leurs sels alcalins ou on fait réagir des halogénures d'acides benzènesulfiniques substitués correspondants ou, en présence d'agents de condensation acides, également des acides sulfiniques substitués correspondants ou leurs sels alcalins avec une N-$R^2$--N'-hydroxy-urée,

on convertit éventuellement les sels obtenus en les composés libres et on traite éventuellement les produits de réaction avec des agents alcalins pour former des sels.

3. Médicament contenant une sulfonylurée selon la revendication 1 ou un de ses sels.

4. Sulfonylurée selon la revendication 1 ou un de ses sels pour lutter contre le diabète.

5. Procédé pour la préparation d'un médicament selon la revendication 3, caractérisé en ce qu'on met une sulfonylurée répondant à la formule indiquée dans la revendication 1, ou un de ses sels, sous une forme d'administration appropriée.

**Revendications pour l'Etat contractant:** AT

1. Procédé pour la préparation d'une sulfonylurée de formule

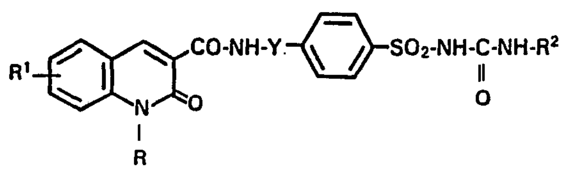

dans laquelle

Y représente un groupe alkylène ayant de 2 à 3 atomes de carbone

R représente un groupe alkyle ayant de 1 à 4 atomes de carbone,

$R^1$ représente l'hydrogène, le chlore, un groupe méthyle,

$R^2$ représente un groupe alkyle ayant de 3 à 6 atomes de carbone, cycloalkyle, alkylcycloalkyle, cycloalkylalkyle, cycloalcényle, alkylcycloalcényle ou cycloalcénylalkyle, ayant chacun de 5 à 8 atomes de carbone,

et ses sels physiologiquement acceptables, lequel procédé est caractérisé en ce que:

a) on fait réagir des dérivés de l'acide benzènesulfonylcarbamique substitué en position 4 avec le groupe

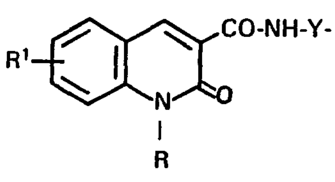

avec une amine $R^2$-$NH_2$ ou ses sels, ou on fait réagir des sulfonamides de formule

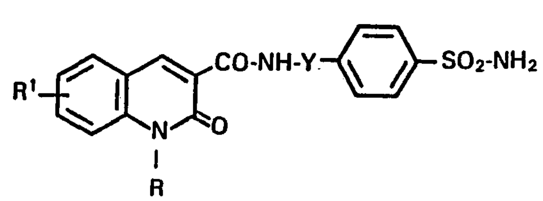

ou leurs sels avec un dérivé de l'acide carbamique $R^2$-substitué;

b) on scinde des éthers de benzènesulfonyl-isourées, des éthers de benzènesulfonylisothiourées, des acides benzènesulfonyl-parabaniques ou des amidines d'acides benzènesulfonyl-halogéno-formiques, tous substitués avec le groupe

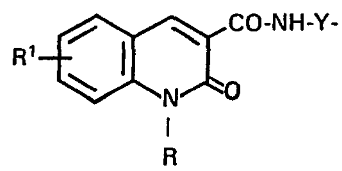

c) dans des benzènesulfonylthiourées substitués avec le groupe

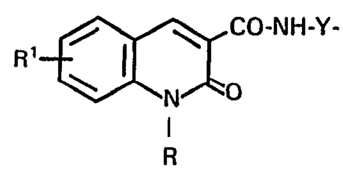

on remplace l'atome de soufre par de l'oxygène;

d) on oxyde des benzènesulfinyl- ou benzènesulfényl-urées correspondantes;

e) dans des benzènesulfonylurées de formule

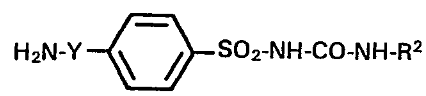

on introduit éventuellement par étapes le radical

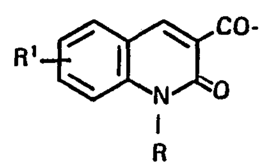

f) on fait réagir des halogénures de benzènesulfonyle substitués correspondants avec des urées $R^2$-substituées ou avec leurs sels alcalins ou on fait réagir des halogénures d'acides benzènesulfiniques substitués correspondants ou, en présence d'agents de condensation acides, également des acides sulfiniques substitués correspondants ou leurs sels alcalins avec une N-$R^2$--N'-hydroxy-urée,

on convertit éventuellement les sels obtenus en les composés libres et on traite éventuellement les produits de réaction avec des agents alcalins pour former des sels.

2. Procédé pour la préparation d'un médicament, caractérisé en ce qu'on met une sulfonylurée répondant à la formule indiquée dans la revendication 1 ou un de ses sels, sous une forme d'administration appropriée, éventuellement en ajoutant des véhicules et adjuvants usuels.